# EUROPÄISCHE PATENTSCHRIFT

(11) **EP 2 326 706 B1**
(45) Veröffentlichungstag und Bekanntmachung des Hinweises auf die Patenterteilung: **04.04.2012**
(21) Anmeldenummer: 09757281.2
(22) Anmeldetag: 03.06.2009
(51) Int. Cl.: C12M 1/00, C12N 1/12

(54) **AUFZUCHT- UND REPRODUKTIONSANLAGE FÜR LICHTINTENSIVE MIKROORGANISMEN (Z.B. ALGEN)**
BREEDING AND REPRODUCTION SYSTEM FOR LIGHT-INTENSIVE MICROORGANISMS (SUCH AS ALGAE)
INSTALLATION POUR LA CULTURE ET LA REPRODUCTION DE MICRO-ORGANISMES (PAR EXEMPLE, DES ALGUES) SOUS LUMIÈRE INTENSIVE

(30) Priorität: 05.06.2008 DE 102008026829
(43) Veröffentlichungstag der Anmeldung: 01.06.2011
(73) Patentinhaber: PanAlgaea Swiss GmbH, 5413 Birmenstorf AG (CH); KURZWEIL, Horst, 97070 Würzburg (DE)
(72) Erfinder: SCHUSTER, Jürgen, 34308 Bad Emstal (DE); KURZWEIL, Horst, 97070 Würzburg (DE)
(74) Vertreter: Finger, Catrin
(86) Internationale Anmeldenummer: PCT/EP2009/003966
(87) Internationale Veröffentlichungsnummer: WO 2009/146898

(56) Entgegenhaltungen:
- WO-A1-00/23562
- WO-A1-02/31102
- DE-A1- 2 358 701
- DE-U1- 20 017 229
- JP-A- 8 140 662
- JP-A- 2001 231 538
- US-B1- 6 287 852

## Beschreibung

Die Erfindung betrifft eine Anlage zur Aufzucht und Reproduktion von Mikroorganismen sowie ein entsprechendes Verfahren dafür.

Die Aufzucht und Reproduktion von Mikroorganismen, insbesondere von Algen, erfolgt üblicherweise in einem offenen Flachbecken, welches eine Höhe von circa 30 cm aufweist.

Eine solche Anlage zur Aufzucht und Reproduktion von Algen ist aus der DE 23 58 701 bekannt, welche ein mit einer Nährstoffsuspension gefülltes Flachbecken aufweist, in welchem sich Trennwände befinden, welche derart angeordnet sind, dass ein Horizontalmäandersystem zur Erzielung eines horizontalen Strömungsweges der Nährstoffsuspension innerhalb des Flachbeckens vorgesehen ist. Zur Erzielung einer Strömungsbewegung der Nährstoffsuspension innerhalb des Flachbeckens ist eine Pumpenanordnung vorgesehen, welche die Nährstoffsuspension in das Flachbecken pumpt.

Durch die geringe Höhe der Flachbecken wird jedoch eine niedrige Volumenausschöpfung des Beckens erreicht, so dass der erreichbare Algenertrag pro Hektar gering ist. Ferner kann es bei einem Flachbecken durch das Verhältnis einer großen Oberfläche zu einem relativ kleinen Volumen des Beckens bzw. der Nährstoffsuspension in dem Becken dazu kommen, dass sich die Nährstoffsuspension innerhalb des Beckens durch die einfallende Lichteinstrahlung zu stark erhitzt, dass es zu einer Schädigung der sich bildenden Mikroorganismen kommen kann. Die Verwendung einer Pumpenanordnung innerhalb des Flachbeckens zur Erzielung einer Strömung innerhalb der Nährstoffsuspension kann zudem aufgrund des dadurch erzeugten relativ hohen Druckes in der Nährstoffsuspension ebenfalls zu einer Schädigung und damit zu einer Verminderung des Wachstums der empfindlichen Mikroorganismen führen.

Aufgabe der Erfindung ist es daher, eine Anlage und ein entsprechendes Verfahren zur Aufzucht und Reproduktion von Mikroorganismen zur Verfügung zu stellen, mit Hilfe welcher ein verbesserter Ertrag an Mikroorganismen pro Hektar erzielbar ist.

Diese Aufgabe wird erfindungsgemäß durch eine Anlage zur Aufzucht und Reproduktion von Mikroorganismen mit den Merkmalen des Anspruchs 1 sowie durch ein entsprechendes Verfahren mit den Merkmalen des Anspruchs 14 gelöst. Vorteilhafte Weiterbildungen der Erfindung sind in den abhängigen Ansprüchen angegeben.

Die Erfindung schließt die technische Lehre ein, dass die Beckenanlage ein durch Trennwände gebildetes Vertikalmäandersystem aufweist, um eine im Wesentlichen vertikale Strömung der Nährstoffsuspension in der Beckenanlage zu erreichen. Durch eine vertikale Strömung innerhalb der Beckenanlage ist es möglich anstelle von Flachbecken Tiefbecken zu verwenden. Die Tiefe der Beckenanlage beträgt dabei vorzugsweise zwischen 1,80 m und 2,20 m. Durch die größere Tiefe der Beckenanlage ist gleichzeitig die Länge der Beckenanlage größer ausgestaltbar als dies bei dem Einsatz von Flachbecken der Fall ist, so dass mit der erfindungsgemäßen Anlage ein verbesserter Ertrag an Mikroorganismen, insbesondere an Algen, pro Hektar benötigter Grundfläche erzielbar ist, wodurch eine optimale Volumenausschöpfung der Beckenanlage erreicht wird. Die im Wesentlichen vertikale Strömung der Nährstoffsuspension in der Beckenanlage führt ferner dazu, dass sich ein für die Aufzucht der Mikroorganismen besonders vorteilhaftes Klima einstellt, da eine Überhitzung der Nährstoffsuspension vermieden werden kann. Zudem wird durch die vertikale Strömung eine besonders gute Durchmischung der Nährstoffsuspension erzielt, wodurch das Wachstum der Mikroorganismen in der Nährstoffsuspension gefördert wird.

Eine vorteilhafte Ausführungsform der Erfindung sieht vor, dass die Beckenanlage mehrere nebeneinander angeordnete Seitenwände aufweisende Becken aufweist, wobei jedes Becken eine Trennwand aufweist und die Seitenwände nebeneinander angeordneter Becken einen Überströmungsbereich der Nährstoffsuspension von einem Becken in das dazu benachbarte Becken bilden. Die Becken sind dabei vorzugsweise U-förmig ausgebildet und weisen eine Breite von circa 2 m bis 3 m, eine Höhe von circa 1,80 m bis 2,20 m und eine Länge von circa 0,2 m bis 0,4 m auf. Dabei sind beliebig viele Becken nebeneinander anordbar, so dass vorzugsweise eine Beckenlänge von mehr als 100 m vorgesehen und problemlos realisierbar ist. Die Strömung innerhalb der Becken erfolgt hauptsächlich vertikal zum Beckenboden entlang der Trennwände. Lediglich im Bereich zwischen dem Beckenboden und dem unteren Rand einer Trennwand sowie im Überströmungsbereich zweier benachbarter Becken, d.h. oberhalb der Seitenwände der Becken, weist die Nährstoffsuspension eine horizontale Strömung auf. Durch die Überströmungsbereiche bleibt die Nährstoffsuspension in Bewegung, so dass keine zusätzliche Pumpenkraft innerhalb der Beckenanlage erforderlich ist, um eine Strömungsbewegung der Nährstoffsuspension innerhalb der Beckenanlage zu bewirken. Die Strömungsbewegung innerhalb der Beckenanlage erfolgt dadurch mit einer relativ geringen Geschwindigkeit und ohne Druck, wodurch die Mikroorganismen innerhalb der Nährstoffsuspension besonders schonend behandelt werden und eine Schädigung der Mikroorganismen während ihres Wachstumsprozesses vermieden wird.

Eine weitere vorteilhafte Ausgestaltung der Erfindung sieht vor, dass die Becken ringförmig angeordnet sind. Durch die ringförmige Anordnung der Becken kann die Nährstoffsuspension von einem Becken zum nächsten Becken strömen, ohne dabei über ein zusätzliches Rückführungssystem von dem letzten Becken zum ersten Becken geleitet werden zu müssen. Zudem ist die Beckenanlage doppelt so groß ausgestaltbar, so dass der Ertrag an Mikroorganismen gesteigert werden kann. Mit der ringförmigen Anordnung der Becken ist daher eine besonders hohe Effektivität der Anlage erzielbar.

Vorteilhafterweise ist eine Hebewerkanordnung zur Einbringung der Nährstoffsuspension in die Beckenanlage vorgesehen, so dass die Nährstoffsuspension aus einem Nährstoffsuspensionsreservoir mittels der Hebewerkanordnung über eine Seitenwand eines ersten Beckens der Beckenanlage in die Beckenanlage einströmen kann. Die Hebewerkanordnung kann dabei in Form einer Platte ausgestaltet sein, die ein außerhalb der Beckenanlage angeordnete Nährstoffsuspensionsreservoir anhebt, so dass Nährstoffsuspension aus dem Reservoir über die Seitenwand des ersten Beckens der Beckenanlage in die Beckenanlage eingebracht wird, indem die Nährstoffsuspension von dem Reservoir über die Seitenwand in das erste Becken überschwappt. Durch dieses Überschwappen wird innerhalb der Nährstoffsuspension in der Beckenanlage eine Strömung erzeugt, welche sich von dem ersten Becken bis zu dem letzten Becken hin fortsetzt. Durch die Verwendung einer Hebewerkanordnung ist es möglich, die Einbringung der Nährstoffsuspension in die Beckenanlage ohne die Aufbringung von Druck, beispielsweise mittels einer Pumpenförderung, durchzuführen, wodurch eine besonders schonende Behandlung der Nährstoffsuspension erreicht wird und dadurch auch die Zellwände der gezüchteten Mikroorganismen besonders schonend behandelt werden, so dass diese bei dem Transport von einem Becken zum nächsten Becken nicht geschädigt werden. Als Hebewerksanordnung haben sich auch Becherförderer als sehr geeignet erwiesen.

Vorzugsweise ist gemäß einer weiteren Ausführungsform vorgesehen, dass die Seitenwand zwischen der Hebewerkanordnung und dem ersten Becken einen Aufsatz aufweist. Durch den Aufsatz ist die Seitenwand zwischen der Hebewerkanordnung und dem ersten Becken höher ausgestaltet, als die restlichen Seitenwände zwischen den einzelnen Becken. Dadurch, dass die Nährstoffsuspension beim Einströmen in die Beckenanlage die Seitenwand und einen darauf angeordneten Aufsatz und damit eine größere Höhe überwinden muss, wird innerhalb der Nährstoffsuspension eine Strömungsbewegung mit einer bestimmten Geschwindigkeit erzielt, ohne dass hierfür eine Pumpenanordnung erforderlich ist.

Eine alternative Ausführungsform zu der Hebewerkanordnung sieht vor, dass die Nährstoffsuspension mittels einer Pumpe in die Beckenanlage eingebracht wird. Es kann jedoch auch vorgesehen sein, dass eine derartige Pumpe zusätzlich zu der Hebewerkanordnung vorgesehen ist, wodurch besonders exakt eine bestimmte Geschwindigkeit der Strömungsbewegung der Nährstoffsuspension einstellbar ist. Mittels der Pumpe ist es auch möglich bei nicht ringförmig angeordneten Becken Nährstoffsuspension aus dem letzten Becken über einen Strömungskanal zurück in das erste Becken zu befördern.

Gemäß einer weiteren Ausführungsform der Erfindung ist die Hebewerkanordnung als Verbindungsrohr zwischen einem letzten Becken und einem ersten Becken ausgebildet. In dem Verbindungsrohr ist eine Förderschnecke mit regel- und/oder steurerbar einstellbarer Drehzahl motorisch antreibbar gelagert. Mittels der Föderschnecke wird in der Suspensionslösung ein Druck aufgebaut, wodurch die Suspensionslösung vom letzten Becken durch das Verbindungsrohr in das erste Becken gefördert wird und somit einen "Flüssigkeitsüberstand" im ersten Becken erzeugt. Hierdurch entsteht die für die Förderung der Suspensionslösung durch die Deckenanlage erforderliche Anhebung des Füllstandes im ersten Becken.

Nach einer weiteren vorteilhaften Ausgestaltung der Erfindung weisen die Trennwände lichtdurchlässige Bereiche auf. Durch die lichtdurchlässigen Bereiche ist es möglich, über die Trennwände, welche vorzugsweise hohl ausgestaltet sind, Licht, Wärme und Energie in die Nährstoffsuspension, insbesondere in die Nährstoffsuspension im Bereich des Beckenbodens, einzubringen, wodurch die Photosynthese und damit das Wachstum der Mikroorganismen in der Nährstoffsuspension gesteigert wird. Die Trennwände können dabei auch über ihre gesamte Umfangsfläche lichtdurchlässig ausgestaltet sein. Die Trennwände bzw. die lichtdurchlässigen Bereiche können dabei beispielsweise aus Milchglas oder einem transparenten Kunststoff gebildet sein. Dadurch, dass über die Trennwände Wärme in die Nährstoffsuspension eingebracht wird, können im Bereich entlang der Trennwand innerhalb der Nährstoffsuspension Konvektionseffekte auftreten, welche Wirbeleffekte innerhalb der Nährstoffsuspension bewirken können, wodurch wiederum eine besonders gute Durchmischung der Nährstoffsuspension erzielt werden kann.

Besonders bevorzugt ist vorgesehen, dass die Trennwände eine dispersive Flüssigkeit aufweisen. Die Trennwände sind dabei im Wesentlichen hohl ausgestaltet und sind mit der dispersiven Flüssigkeit gefüllt. Mittels der dispersiven Flüssigkeit ist es möglich, das Licht, die Wärme und auch die Energie auf einfache Art und Weise innerhalb der Trennwände einzuleiten, in die Nährstoffsuspension einzubringen und gleichmäßig darin zu verteilen. Die dispersive Flüssigkeit enthält dabei dispersive Partikel, die wie Lichtableiter wirken und dadurch eine besonders effektive Einbringung des Lichts in die Nährstoffsuspension mit einem hohen Wirkungsgrad erreichen. Die dispersive Flüssigkeit kann dabei aus einer transparenten Flüssigkeit, wie Wasser, bestehen, welche nicht gelöste Farbpigmente enthält. Dadurch, dass die Trennwände vorzugsweise vollständig mit der dispersiven Flüssigkeit befüllt sind, befindet sich innerhalb der Trennwände eine große Fluidmenge, die auf Temperaturschwankungen sehr träge reagiert, wodurch es möglich ist, eine nahezu konstante Temperatur und damit eine nahezu konstante Energie- und Wärmeeinbringung in die Nährstoffsuspension zur Verfügung zu stellen.

Gemäß einer weiteren vorteilhaften Ausführungsform weisen die Trennwände eine Röhrenanordnung auf, durch welche die dispersive Flüssigkeit geleitet werden kann. Die Röhrenanordnung erstreckt sich dabei vorzugsweise über die gesamte Längsseite der Trennwände in Form einer Röhrenschlange. Die dispersive Flüssigkeit wird dabei durch die Röhrenanordnung mit einer bestimmten Geschwindigkeit möglichst gleichmäßig geleitet, so dass eine besonders homogene Temperierung der Beckenanlage und damit der Nährstoffsuspension innerhalb der Beckenanlage ermöglicht wird.

Vorzugsweise weisen die Trennwände eine Leuchtdiodenanordnung auf, mittels welcher Licht, Energie und Wärme in die Nährstoffsuspension eingebracht werden kann. Die Leuchtdiodenanordnung ist dabei vorzugsweise im unteren Bereich der Trennwände im Bereich des Beckenbodens angeordnet, so dass auch in diesem Bereich der Beckenanlage noch ausreichend Licht in die Nährstoffsuspension eingebracht werden kann. Die Leuchtdiodenanordnung zeichnet sich durch eine besonders hohe Lebensdauer aus. Vorzugsweise werden Leuchtdioden mit einer Leistung von 100 W eingesetzt, um eine höhere Abwärme erzielen zu können. Anstelle von Leuchtdioden können jedoch auch übliche Leuchtmittel, wie Glühbirnen, verwendet werden.

Ferner können die Trennwände vorteilhafterweise Lichtsammler zur Bündelung des Sonnenlichtes aufweisen. Die Lichtsammler sind dabei vorzugsweise im oberen Bereich der Trennwände au-βerhalb der Becken angeordnet. Die Lichtsammler sammeln und bündeln das Sonnenlicht aus der Umgebung und leiten dieses in die Trennwände ein. Dabei kann jede Trennwand einen separaten Lichtsammler aufweisen. Dieses gebündelte Sonnenlicht weist einen besonders hohen Lichtenergieanteil und einen hohen Wärmeanteil auf, welche über die Trennwände an die Nährstoffsuspension abgegeben werden können. Dadurch kann die Einbringung an Energie und Wärme in die Nährstoffsuspension gesteigert werden, wodurch auf einfache Art und Weise und mit geringen Kosten die Photosynthese und damit das Wachstum der Mikroorganismen verbessert werden kann. Die Lichtsammler können in Form optischer Vorrichtungen, wie Sammellinsen, ausgebildet sein.

Dadurch, dass nach einer weiteren Ausführungsform die Trennwände Heizelemente und/oder Kühlelemente aufweisen, ist es möglich, Temperaturschwankungen möglichst schnell ausgleichen zu können, um eine möglichst optimale Temperaturverteilung innerhalb der Trennwände und damit innerhalb der Nährstoffsuspension einstellen zu können, so dass ein optimales Klima zur Aufzucht der Mikroorganismen geschaffen werden kann.

Gemäß einer weiteren Ausführungsform der Erfindung ist zur Temperaturregelung der Suspensionslösung vorgesehen, Heiz-und/oder Kühlelemente in oder an den Beckenwänden und in oder an den Mäanderwänden anzubringen. Die Heiz- und oder Kühlelemente können sowohl zur Beckeninnenseite hinweisen, an den Beckenwänden oder den Mäanderwänden. Gleichfalls ist es selbstverständlich möglich die Heiz- und/oder Kühlelemente außenseitig an den Beckenwänden anzuordnen. Dies hat den Vorteil, dass die Wärmeleitung durch die Beckenwände hindurch erfolgen muss, was einen geringeren und somit für die Suspensionslösung schonenderen Temperaturgradienten zur Folge hat.

Insbesondere ist es vorteilhaft, die erforderliche Heiz-und/oder Kühlenergie durch das Vorbeileiten der Dispersionsflüssigkeit an Wärmetauscherflächen mittels Pumpen zu gewährleisten.

Eine weitere vorteilhafte Ausgestaltung der Erfindung sieht vor, dass die Trennwände über einen Steg miteinander verbunden sind. Der Steg ist dabei am oberen Ende der Trennwände oberhalb der Nährstoffsuspension angeordnet. Der Steg ist vorzugsweise hohl ausgestaltet und über den Steg kann die dispersive Flüssigkeit von einer Trennwand zu nächsten Trennwand fließen, so dass ein permanenter Austausch an dispersiver Flüssigkeit zwischen den Trennwänden stattfindet. In dem Steg können Heizelemente und/oder Kühlelemente angeordnet sein, mit Hilfe welcher eine für das Wachstum der Mikroorganismen optimale Temperatur einstellbar ist. Der Steg ist bevorzugt oberhalb der Beckenanlage angeordnet und kann dieser als Lichtsammler des Sonnenlichts dienen, über welchen das gesammelte Sonnelicht an die einzelnen Trennwände abgegeben wird.

Die Erfindung betrifft ferner ein Verfahren zur Aufzucht und Reproduktion von Mikroorganismen mittels einer wie vorstehend aus- und weitergebildeten Anlage, bei dem Licht in eine eine Nährstoffsuspension enthaltende Beckenanlage eingebracht wird, wobei die Einbringung des Lichts über in die Nährstoffsuspension hineinragende Trennwände erfolgt, welche mit einer dispersiven Flüssigkeit gefüllt sind.

Mittels der dispersiven Flüssigkeit ist es möglich, das zur Aufzucht der Mikroorganismen erforderliche Licht und die erforderliche Wärme möglichst effektiv auf einfache Art und Weise in die Nährstoffsuspension einzubringen und gleichmäßig darin zu verteilen.

Die dispersive Flüssigkeit enthält dabei vorzugsweise dispersive Partikel, die wie Lichtableiter wirken und dadurch eine besonders effektive Einbringung des Lichts in die Nährstoffsuspension bewirken. Die dispersive Flüssigkeit kann dabei aus Wasser bestehen, welches nicht gelöste Farbpigmente enthält. Die Trennwände sind vorzugsweise vollständig von der dispersiven Flüssigkeit ausgefüllt, so dass sich innerhalb der Trennwände eine große Fluidmenge befindet, die auf Temperaturschwankungen sehr träge reagiert. Hierdurch ist es möglich ist, eine nahezu konstante Temperatur und damit eine nahezu konstante Energie- und Wärmeeinbringung in die Nährstoffsuspension zur Verfügung zu stellen.

Um eine möglichst homogene und leicht einstellbare Temperierung der Trennwände über ihre gesamte Längsseite zu erzielen, ist es vorzugsweise vorgesehen, dass die dispersive Flüssigkeit durch eine in den Trennwänden angeordnete Röhrenanordnung fließt.

In Bezug auf die Vorteile des erfindungsgemäßen Verfahrens wird ferner vollumfänglich auf die erfindungsgemäße Anlage zur Aufzucht und Reproduktion von Mikroorganismen verwiesen.

Nachfolgend wird die Erfindung unter Bezugnahme auf die anliegenden Zeichnungen anhand bevorzugter Ausführungsbeispiele näher erläutert.

Es zeigen:
- Fig. 1: eine schematische Darstellung eines ersten Ausführungsbeispiels einer erfindungsgemäßen Anlage zur Aufzucht und Reproduktion von Mikroorganismen;
- Fig. 2: eine schematische Darstellung eines zweiten Ausführungsbeispiels einer erfindungsgemäßen Anlage zur Aufzucht und Reproduktion von Mikroorganismen;
- Fig. 3: eine schematische Darstellung eines Ausführungsbeispiels einer erfindungsgemäßen Trennwand;
- Fig. 4: eine schematische Darstellung eines Ausführungsbeispiels einer erfindungsgemäßen Beckenanordnung und
- Fig. 5: eine schematische Darstellung eines dritten Ausführungsbeispiels einer erfindungsgemäßen Anlage zur Aufzucht und Reproduktion von Mikroorganismen

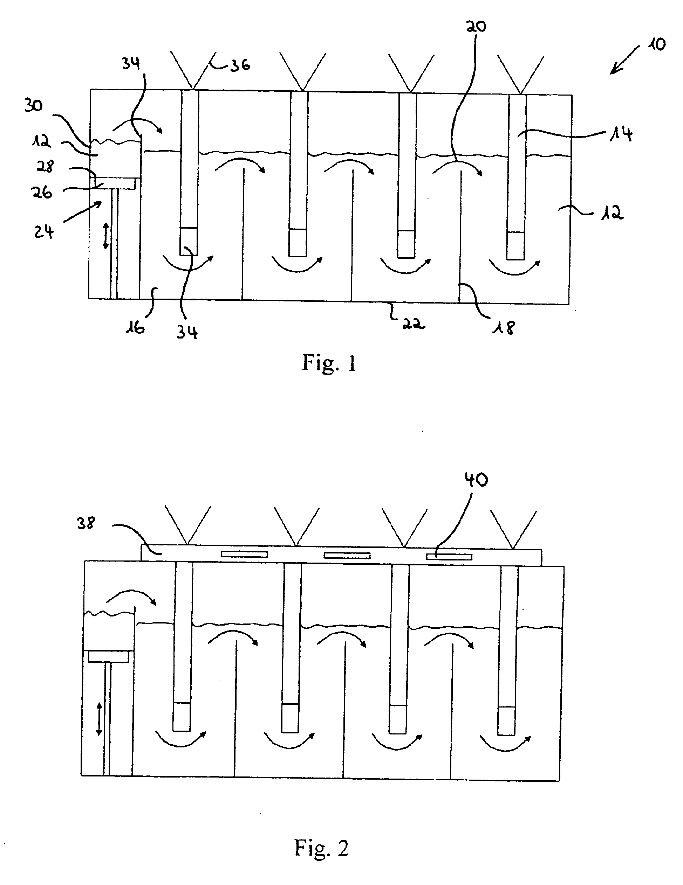

In Fig. 1 ist eine schematische Darstellung eines ersten Ausführungsbeispiels einer erfindungsgemäßen Anlage zur Aufzucht und Reproduktion von Mikroorganismen gezeigt, mit einer Beckenanlage 10 und einer in der Beckenanlage angeordneten Nährstoffsuspension 12, wobei die Beckanlage 10 ein durch Trennwände 14 gebildetes Vertikalmäandersystem aufweist, um eine im Wesentlichen vertikale Strömung der Nährstoffsuspension 12 in der Beckenanlage 10 zu erreichen. Die Beckenanlage 10 ist aus mehreren nebeneinander angeordneten, offenen, U-förmigen Becken 16 aufgebaut, wobei in jedes Becken 16 eine Trennwand 14 eingetaucht ist. Jedes Becken 16 weist Seitenwände 18 auf, wobei die Seitenwände 18 nebeneinander angeordneter Becken 16 einen Überströmungsbereich 20 der Nährstoffsuspension 12 von einem Becken 16 in das dazu benachbarte Becken bilden. Innerhalb der Beckenanlage 10 folgt die Nährstoffsuspension 12 im Wesentlichen einer durch Pfeile angedeuteten vertikalen Strömung im Bereich zwischen einer Seitenwand 18 und einer Trennwand 14. Im Überströmungsbereich 20 und im Bereich zwischen dem Beckenboden 22 und dem unteren Ende der Trennwand 14 wird die Strömung, wie durch die Pfeile gezeigt, umgelenkt, so dass hierdurch ein Vertikalmäandersystem gebildet wird.

Zur Einbringung der Nährstoffsuspension 12 in die Beckenanlage 10 ist eine Hebewerkanordnung 24 vorgesehen. Die Hebewerkanordnung weist eine auf und ab bewegbare Platte 26 auf, mit Hilfe welcher die Bodenplatte 28 eines Nährstoffsuspensionsreservoirs 30 nach oben bewegbar ist, so dass Nährstoffsuspension 12 über den Rand des Reservoirs 30 über die Seitenwand 32 eines ersten Beckens 16 der Beckenanlage 10 überschwappen und dadurch in die Beckenanlage 10 einströmen kann. Durch diese Anordnung wird erreicht, dass die Nährstoffsuspension 12 ohne Druck möglichst schonend in die Beckenanlage 10 eingebracht wird und dabei gleichzeitig innerhalb der Beckenanlage 10 eine leichte Strömung erzielt wird. Die Seitenwand 18 zwischen dem Reservoir 30 und dem ersten Becken 16 weist dabei einen Aufsatz 34 auf, so dass die Höhe dieser Seitenwand 32 höher ausgestaltet ist, als die Höhe der übrigen in der Beckenanlage 10 angeordneten Seitenwände 18.

Die Trennwände 14 sind möglichst mittig in die Becken 16 eingetaucht. Die Trennwände 14 weisen lichtdurchlässige Bereiche auf, über welche Licht, Energie und Wärme in die Nährstoffsuspension eingebracht werden kann. Die lichtdurchlässigen Bereiche können auch über die gesamte Umfangsfläche der Trennwände 14 ausgebildet sein. Die Trennwände 14 bzw. die lichtdurchlässigen Bereiche können dabei beispielsweise aus Milchglas oder einem transparenten Kunststoff ausgestaltet sein.

Die Trennwände 14 sind vorzugsweise hohl ausgestaltet. Innerhalb der Trennwände ist eine dispersive Flüssigkeit angeordnet, über welche auf einfache Art und Weise Licht, Energie und Wärme in den Trennwänden gespeichert und an die Nährstoffsuspension 12 abgegeben werden kann. Die dispersive Flüssigkeit weist dispersive Partikel auf, die wie Lichtableiter wirken und dadurch eine besonders effektive Einbringung des Lichts in die Nährstoffsuspension 12 erzielen. Die dispersive Flüssigkeit kann dabei aus Wasser bestehen, welches nicht gelöste Farbpigmente enthält. Dadurch, dass die Trennwände 14 vorzugsweise vollständig von der dispersiven Flüssigkeit ausgefüllt sind, befindet sich innerhalb der Trennwände 14 eine große Fluidmenge, die auf Temperaturschwankungen sehr träge reagiert. Hierdurch ist es möglich, eine nahezu konstante Temperatur und damit eine nahezu konstante Energie- und Wärmeeinbringung in die Nährstoffsuspension 12 zur Verfügung zu stellen.

Die Trennwände 14 weisen eine Leuchtdiodenanordnung 34 auf, welche bevorzugt am unteren Ende der Trennwände 14 angeordnet sind. Durch die Leuchtdiodenanordnung 34 wird zusätzlich Licht und Wärme in die Nährstoffsuspension 12 eingebracht. Ferner weisen die Trennwände 14 Lichtsammler 36 auf, welche im oberen Bereich der Trennwände 14, oberhalb der Nährstoffsuspension 12 bzw. der Beckenanlage 10 angeordnet sind. Die Lichtsammler 36 sammeln und bündeln das einfallende Sonnenlicht und geben dieses an die in den Trennwänden 14 angeordnete dispersive Flüssigkeit ab, über welche die Energie, die Wärme und das Licht des Sonnelichts wiederum an die Nährstoffsuspension 12 in der Beckenanlage 10 abgegeben wird.

Um die Temperatur innerhalb der Trennwände 14 optimal einstellen zu können, können die Trennwände 14 ferner hier nicht dargestellte Heiz- und/oder Kühlelemente aufweisen.

Dadurch, dass über die Trennwände 14 Wärme in die Nährstoffsuspension eingebracht wird, können im Bereich entlang der Trennwände 14 innerhalb der Nährstoffsuspension 12 Konvektionseffekte auftreten, welche Wirbeleffekte innerhalb der Nährstoffsuspension 12 hervorrufen können, wodurch wiederum eine besonders gute Durchmischung der Nährstoffsuspension 12 erzielt werden kann.

Wie in Fig. 2 gezeigt, können die Trennwände 14 gemäß eines zweiten Ausführungsbeispiels über einen Steg 38 miteinander verbunden sein, welcher hohl ausgestaltet ist und in welchem ebenfalls dispersive Flüssigkeit angeordnet ist. Über den Steg 38 kann die dispersive Flüssigkeit von einer Trennwand 14 zur nächsten Trennwand fließen, so dass ein permanenter Austausch an dispersiver Flüssigkeit zwischen den Trennwänden 14 stattfinden kann. In dem Steg 38 können Heizelemente und/oder Kühlelemente 40 angeordnet sein, mit Hilfe welcher eine für das Wachstum der Mikroorganismen optimale Temperatur einstellbar ist. Da der Steg 38 bevorzugt oberhalb der Beckenanlage 10 angeordnet ist, kann dieser als Lichtsammler des Sonnenlichts dienen, wobei das gesammelte Sonnelicht über die dispersive Flüssigkeit an die einzelnen Trennwände 14 abgegeben wird.

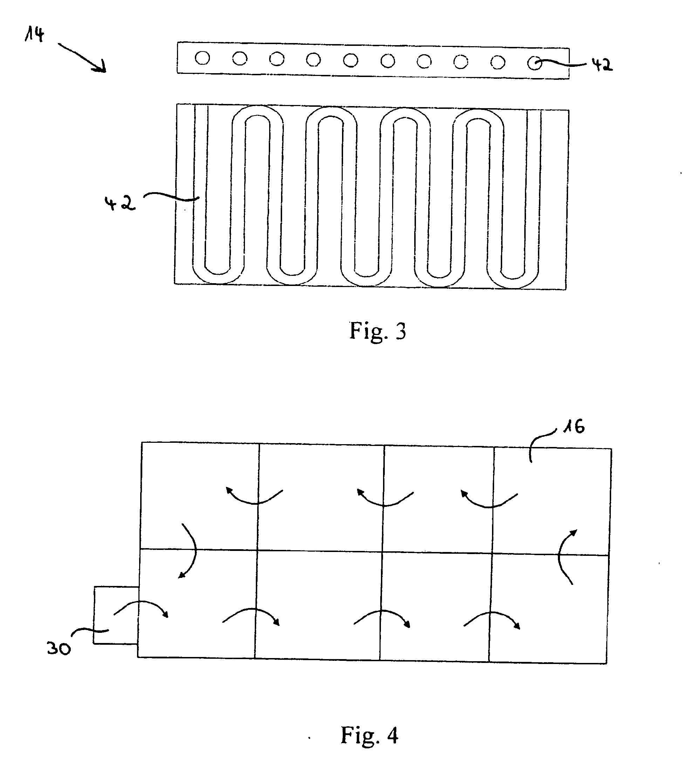

In Fig. 3 ist schematisch ein Ausführungsbeispiel einer Trennwand 14 dargestellt, welche eine Röhrenanordnung 42 aufweist, durch welche die dispersive Flüssigkeit innerhalb der Trennwände 14 geführt werden kann, so dass eine gleichmäßige Temperaturverteilung entlang der Trennwand 14 ermöglicht wird. Die Röhrenanordnung 42 ist dabei vorzugsweise über die gesamte Längsseite der Trennwand 14 schlangenförmig innerhalb der Trennwand 14 angeordnet.

Um eine einfache und effektive Ausnutzung der Beckenanlage 10 zu ermöglichen, können die Becken 16, wie in Fig. 4 dargestellt, ringförmig angeordnet sein, so dass die Nährstoffsuspension 12 von dem letzten Becken in das erste Becken einfach überströmen kann, ohne dass die Nährstoffsuspension 12 auf umständliche Art und Weise über eine zusätzliche Anlage zurückgeführt werden muss.

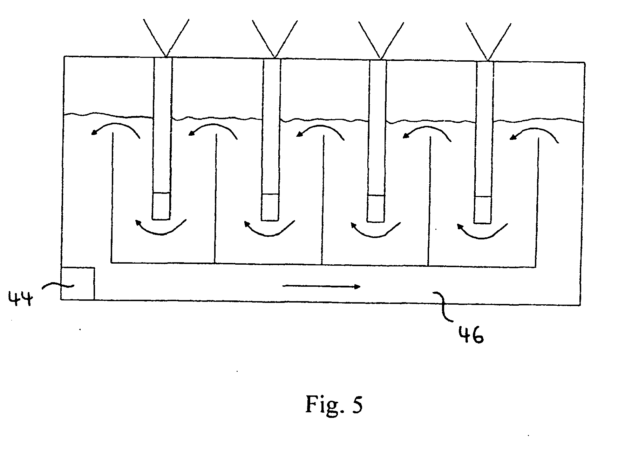

Fig. 5 zeigt eine schematische Darstellung eines dritten Ausführungsbeispiels einer erfindungsgemäßen Anlage, bei der die Nährstoffsuspension 12 mittels einer Pumpe 44 in die Beckenanlage 10 eingebracht wird. Dabei ist unterhalb der einzelnen Becken ein Strömungskanal 46 angeordnet, über welchen die Nährstoffsuspension 12 in die einzelnen Becken 16 gefördert wird. Nach Austritt der Nährstoffsuspension 12 aus dem letzten Becken, wird diese mittels der Pumpe wieder über den Strömungskanal 46 zurück in der erste Becken gepumpt, so dass dadurch ein Strömungskreislauf der Nährstoffsuspension entsteht.

## Patentansprüche

1. Anlage zur Aufzucht und Reproduktion von Mikroorganismen, mit einer Beckenanlage (10) und einer in der Beckenanlage (10) angeordneten Nährstoffsuspension (12), wobei die Beckenanlage (10) ein durch zumindest teilbereichsweise lichtdurchlässige Trennwände (14) gebildetes Vertikalmäandersystem aufweist, um eine im Wesentlichen vertikale Strömung der Nährstoffsuspension (12) in der Beckenanlage (10) zu erreichen, **dadurch gekennzeichnet, dass** die Trennwände (14) hohl ausgebildet sind und eine dispersive Flüssigkeit zum Ableiten von Licht in die Nährstoffsuspension aufweisen.

2. Anlage nach Anspruch 1, **dadurch gekennzeichnet, dass** die Beckenanlage (10) mehrere nebeneinander angeordnete Seitenwände (18) aufweisende Becken (16) zumindest aufweist, wobei jedes Becken (16) eine Trennwand (14) aufweist und die Seitenwände (18) nebeneinander angeordneter Becken (16) einen Überströmungsbereich (20) der Nährstoffsuspension (12) von einem Becken (16) in das dazu benachbarte Becken bilden.

3. Anlage nach Anspruch 2, **dadurch gekennzeichnet, dass** die Becken (16) ringförmig angeordnet sind.

4. Anlage nach einem der Ansprüche 1 bis 3, **dadurch gekennzeichnet, dass** eine Hebewerkanordnung (24) zur Einbringung der Nährstoffsuspension (12) in die Beckenanlage (10) vorgesehen ist, so dass Nährstoffsuspension (12) aus einem Nährstoffsuspensionsreservoir (30) mittels der Hebewerkanordnung (24) über eine Seitenwand (18) eines ersten Beckens (16) der Beckenanlage (10) in die Beckenanlage (10) einströmen kann.

5. Anlage nach Anspruch 4, **dadurch gekennzeichnet, dass** die Seitenwand (32) zwischen der Hebewerkanordnung (24) und dem ersten Becken (16) einen Aufsatz (34) aufweist.

6. Anlage nach Anspruch 4 und/oder 5, **dadurch gekennzeichnet, dass** die Hebewerksanordnung ein Verbindungsrohr zwischen dem ersten Becken (16), der Beckenanlage (10) und einem letzen Becken der Beckenanlage (10) ist, durch welches die Nährstoffsuspension (12) fließen kann.

7. Anlage nach Anspruch 6, **dadurch gekennzeichnet, dass** im Verbindungsrohr eine Förderschnecke angeordnet ist, welche insbesondere regel- und/oder steuerbar motorisch antreibbar ist.

8. Anlage nach einem der Ansprüche 1 bis 3, **dadurch gekennzeichnet, dass** die Nährstoffsuspension (12) mittels einer Pumpe (44) in die Beckenanlage (10) eingebracht wird.

9. Anlage nach Anspruch 1, **dadurch gekennzeichnet, dass** die Trennwände (14) eine Röhrenanordnung (42) aufweisen, durch welche die dispersive Flüssigkeit geleitet werden kann.

10. Anlage nach einem der Ansprüche 1 bis 9, **dadurch gekennzeichnet, dass** die Trennwände (14) eine Leuchtdiodenanordnung (34) aufweisen.

11. Anlage nach einem der Ansprüche 1 bis 10, **dadurch gekennzeichnet, dass** die Trennwände (14) Lichtsammler (36) zur Sammelung und Bündelung des Sonnenlichts aufweisen.

12. Anlage nach einem der Ansprüche 1 bis 11, **dadurch gekennzeichnet, dass** die Trennwände (16) Heizelemente und/oder Kühlelemente aufweisen.

13. Anlage nach einem der Ansprüche 1 bis 12, **dadurch gekennzeichnet, dass** die Heizelemente und/oder die Kühlelemente in oder an Beckenaußenwänden der Becken angeordnet sind.

14. Anlage nach einem der Ansprüche 1 bis 13, **dadurch gekennzeichnet, dass** die Heiz- und/oder die Kühlelemente beckenaußenseitig an den Begrenzungswandungen angeordnet sind.

15. Anlage nach einem der Ansprüche 1 bis 14, **dadurch gekennzeichnet, dass** die Trennwände (16) über einen Steg (38) miteinander verbunden sind.

16. Verfahren zur Aufzucht und Reproduktion von Mikroorganismen mittels einer Anlage nach einem der Ansprüche 1 bis 15, bei dem Licht in eine eine Nährstoffsuspension enthaltende Beckenanlage eingebracht wird, **dadurch gekennzeichnet, dass** die Einbringung des Lichts über in die Nährstoffsuspension hineinragende Trennwände erfolgt, welche mit einer dispersiven Flüssigkeit gefüllt sind.

17. Verfahren nach Anspruch 16, **dadurch gekennzeichnet, dass** die dispersive Flüssigkeit lichtableitende Partikel aufweist.

18. Verfahren nach einem der Ansprüche 16 oder 17, **dadurch gekennzeichnet, dass** die dispersive Flüssigkeit durch eine in den Trennwänden angeordnete Röhrenanordnung fließt.

## Claims

1. Plant for breeding and reproducing microorganisms, having a basin system (10) and a nutrient suspension (12) placed in the basin system (10), while the basin system (10) presents a vertical meander system formed by divider baffles (14) transparent at least in subareas in order to achieve an essentially vertical flow of the nutrient suspension (12) in the basin system (10), **characterized in that** the divider baffles (14) have a hollow design, and present a dispersive liquid for diverting light into the nutrient suspension.

2. Plant as claimed in claim 1, **characterized in that** the basin system (10) at least presents basins (16) presenting several side walls (18) located side by side while every basin (16) presents a divider baffle (14), and the side walls (18) of basins (16) located side by side present an overflow area (20) of the nutrient suspension (12) of one basin (16) into its neighbouring basin.

3. Plant as claimed in claim 2, **characterized in that** the basins (16) are arranged in ring form.

4. Plant as claimed in any claim 1 through 3, **characterized in that** a lift arrangement (24) is provided for introducing the nutrient suspension (12) into the basin system (10) by which nutrient suspension (12) may flow from a nutrient suspension reservoir (30) via a side wall (18) of a first basin (16) of the basin system (10) into the basin system (10), using the lift arrangement.

5. Plant as claimed in claim 4, **characterized in that** the side wall (32) between the lift arrangement (24) and the first basins (16) has a top unit (34).

6. Plant as claimed in claim 4 and/or 5, **characterized in that** the lift arrangement is a connecting pipe between the first basin (16), the basin system (10) and a last basin of the basin system (10) through which connecting pipe the nutrient suspension (12) may flow.

7. Plant as claimed in claim 6, **characterized in that** a conveyor worm is located in the connecting pipe and is, in particular, capable of being driven by a motor in a regulable and/or controllable way.

8. Plant as claimed in any claim 1 through 3, **characterized in that** the nutrient suspension (12) is introduced into the basin system (10) by using a pump (44).

9. Plant as claimed in claim 1, **characterized in that** the divider baffles (14) present a tube arrangement (42) through which the dispersive liquid may be conducted.

10. Plant as claimed in any claim 1 through 9, **characterized in that** the divider baffles (14) present a light-emitting diode array (34).

11. Plant as claimed in any claim 1 through 10, **characterized in that** the divider baffles (14) present light collectors (36) for the collection and bundling of sunlight.

12. Plant as claimed in any claim 1 through 11, **characterized in that** the divider baffles (16) present heating elements and/or cooling elements.

13. Plant as claimed in any claim 1 through 12, **characterized in that** the heating elements and/or the cooling elements are arranged in or at outer basin walls of the basins.

14. Plant as claimed in any claim 1 through 13, **characterized in that** the heating and/or cooling elements are arranged at the basin outside at the boundary walls.

15. Plant as claimed in any claim 1 through 14, **characterized in that** the divider baffles (16) are connected to one another using a web (38).

16. Procedure for breeding and reproducing microorganisms using a plant as claimed in any claim 1 through 15, by which procedure light is introduced into a basin system containing a nutrient suspension, **characterized in that** the introduction of the light is ensured via divider baffles which project into the nutrient suspension and which are filled with a dispersive liquid.

17. Procedure as claimed in claim 16, **characterized in that** the dispersive liquid presents light diverting particles.

18. Procedure as claimed in any claim 16 or 17, **characterized in that** the dispersive liquid flows through a tube arrangement arranged in the divider baffles.

## Revendications

1. Installation pour la culture et la reproduction de micro-organismes, avec un système de bassins (10) et une suspension nutritive (12) contenue dans le système de bassins (10), le système de bassins (10) comportant un système de méandres verticaux formé par des cloisons (14) au moins partiellement transparentes, destiné à permettre un écoulement sensiblement vertical de la suspension nutritive (12) dans le système de bassins (10), **caractérisée en ce que** les cloisons (14) sont creuses et comprennent un liquide dispersif pour la diffusion de lumière dans la suspension nutritive.

2. Installation selon la revendication 1, **caractérisée en ce que** le système de bassins (10) comprend plusieurs bassins (16) comportant des parois latérales (18) disposées côte à côte, chaque bassin (16) contenant une cloison (14) et les parois latérales (18) de bassins (16) adjacents formant une zone de déversement (20) de la suspension nutritive (12) d'un bassin (16) dans le bassin contigu.

3. Installation selon la revendication 2, **caractérisée en ce que** les bassins (16) sont disposés en anneau.

4. Installation selon l'une des revendications 1 à 3, **caractérisée en ce qu'**il est prévu un dispositif élévateur (24) destiné à injecter la suspension nutritive (12) dans le système de bassins (10), de telle manière que la suspension nutritive (12) puisse au moyen du dispositif élévateur (24) s'écouler d'un réservoir de suspension nutritive (30) dans le système de bassins (10), au-dessus d'une paroi latérale (18) d'un premier bassin (16) du système de bassins (10).

5. Installation selon la revendication 4, **caractérisée en ce que** la paroi latérale (32) présente une surélévation (34) entre le dispositif élévateur (24) et le premier bassin (16).

6. Installation selon la revendication 4 et/ou la revendication 5, **caractérisée en ce que** le dispositif élévateur est une conduite de connexion entre le premier bassin (16), le système de bassins (10) et un dernier bassin du système de bassins (10), au travers duquel la suspension nutritive (12) peut s'écouler.

7. Installation selon la revendication 6, **caractérisée en ce qu'**une vis de transport est disposée dans la conduite de connexion, laquelle peut être entraînée par moteur, notamment au moyen d'une régulation et/ou d'une commande.

8. Installation selon l'une des revendications 1 à 3, **caractérisée en ce que** la suspension nutritive (12) est injectée dans le système de bassins (10) au moyen d'une pompe (44).

9. Installation selon la revendication 1, **caractérisée en ce que** les cloisons (14) comprennent un système de tubes (42) permettant de conduire le liquide dispersif.

10. Installation selon l'une des revendications 1 à 9, **caractérisée en ce que** les cloisons (14) comprennent un système de diodes électroluminescentes (34).

11. Installation selon l'une des revendications 1 à 10, **caractérisée en ce que** les cloisons (14) comprennent des collecteurs de lumière (36) pour recevoir et concentrer la lumière solaire.

12. Installation selon l'une des revendications 1 à 11, **caractérisée en ce que** les cloisons (16) comprennent des éléments de chauffage et/ou des éléments de refroidissement.

13. Installation selon l'une des revendications 1 à 12, **caractérisée en ce que** les éléments de chauffage et/ou les éléments de refroidissement sont disposés dans ou contre les parois extérieures des bassins.

14. Installation selon l'une des revendications 1 à 13, **caractérisée en ce que** les éléments de chauffage et/ou les éléments de refroidissement sont disposés à l'extérieur des bassins sur les parois de délimitation.

15. Installation selon l'une des revendications 1 à 14, **caractérisée en ce que** les cloisons (16) sont reliées l'une à l'autre par une traverse (38).

16. Procédé pour la culture et la reproduction de micro-organismes au moyen d'une installation selon l'une des revendications 1 à 15, où de la lumière est projetée dans un système de bassins contenant une suspension nutritive, **caractérisé en ce que** la lumière est projetée par des cloisons pénétrant dans la suspension nutritive et qui sont remplies d'un liquide dispersif.

17. Procédé selon la revendication 16, **caractérisé en ce que** le liquide dispersif contient des particules diffusant la lumière.

18. Procédé selon l'une des revendications 16 ou la revendication 17, **caractérisé en ce que** le liquide dispersif s'écoule par un système de tubes disposé dans les cloisons.
